# EUROPÄISCHE PATENTSCHRIFT

(11) **EP 1 986 558 B1**
(45) Veröffentlichungstag und Bekanntmachung des Hinweises auf die Patenterteilung: **10.09.2014**
(21) Anmeldenummer: 07703442.9
(22) Anmeldetag: 13.02.2007
(51) Int. Cl.: A61B 18/14, A61B 17/00, A61B 18/00

(54) **SONDENMANIPULATOR**
PROBE MANIPULATOR
MANIPULATEUR DE SONDE

(30) Priorität: 24.02.2006 DE 102006008739
(43) Veröffentlichungstag der Anmeldung: 05.11.2008
(73) Patentinhaber: ERBE Elektromedizin GmbH, 72072 Tübingen (DE)
(72) Erfinder: SCHNITZLER, Uwe, 72074 Tübingen (DE); HAGG, Martin, 72827 Wannweil (DE)
(74) Vertreter: Bohnenberger, Johannes
(86) Internationale Anmeldenummer: PCT/EP2007/001248
(87) Internationale Veröffentlichungsnummer: WO 2007/098857

(56) Entgegenhaltungen:
- US-A- 4 726 369
- US-A- 4 829 999
- US-A- 5 720 745
- US-A1- 2002 103 418
- US-B1- 6 477 402

## Beschreibung

Die Erfindung betrifft einen Sondenmanipulator.

Bei endoskopischen Operationen werden oftmals Sonden in einen Arbeitskanal eines Endoskops derart eingeführt, dass ihre distalen Enden im Blickfeld der Endoskop-Optik liegen, um mittels der Sonde auf das Gewebe eines Patienten einwirken zu können. Eine derartige Sonde ist beispielsweise aus der US 5,720,745 bekannt und dient zur Koagulation von Gewebe mittels hochfrequenten Stromes, der über ein Argon-Plasma dem Gewebe zugeführt wird. Zum Bewegen der Sonde innerhalb des Endoskopes bzw. dessen Arbeitskanals fasst der Operateur die Sonde nahe der Zugangsöffnung für den Arbeitskanal des Endoskops an. Da die Sonden sehr dünn sind, erfordert diese Vorgehensweise sehr viel "Fingerspitzengefühl".

Aus der US 5,207,675 ist eine APC-Sonde bekannt, an deren proximalem Ende ein Handgriff befestigt ist, um die Sonde innerhalb des Arbeitskanals zu bewegen. Damit aber muss die Sonde passend zu dem Endoskop gefertigt sein, mit welchem sie zu verwenden ist.

Dokument US 2002/103418 A offenbart einen Manipulator mit einer von einem Benutzer ergreifbaren Griffeinrichtung, zum Manipulieren eines Endoskops, mit einer Verbindungseinrichtung zum lösbaren Verbinden des Endoskops mit der Griffeinrichtung derart, dass das Endoskop beim Drehen oder Verschieben der Griffeinrichtung gedreht bzw. verschoben wird, und wobei an der Griffeinrichtung Schalteinrichtungen vorgesehen sind. Das distale Ende des Endoskops kann mit den Schalteinrichtungen gebogen, gedreht oder verschoben werden.

Der Erfindung liegt die Aufgabe zu Grunde, einen Sondenmanipulator aufzuzeigen, der dem Operateur eine präzise und einfache Handhabung der Sonde ermöglicht.

Diese Aufgabe wird durch einen Sondenmanipulator nach Patentanspruch 1 gelöst.

Insbesondere wird die Aufgabe durch einen Sondenmanipulator mit einer, von einem Benutzer ergreifbaren Griffeinrichtung zum Manipulieren einer Sonde, die in einem

Arbeitskanal eines Endoskops eingeführt ist gelöst, wobei eine Verbindungseinrichtung zum lösbaren Verbinden der Sonde mit der Griffeinrichtung derart vorgesehen ist, dass die Sonde beim Drehen oder Verschieben der Griffeinrichtung gedreht bzw. verschoben wird.

Ein wesentlicher Punkt der Erfindung liegt darin, dass nicht die Sonde selbst mit einem Handgriff versehen ist, sondern dass vielmehr ein gesonderter Sondenmanipulator vorgesehen ist, der zunächst getrennt von der Sonde handhabbar, mit dieser aber verbindbar ist, um dann die Sonde während der Arbeit manipulieren zu können. Dadurch kann zum einen der Manipulator mit verschiedenen Sonden, die je nach dem momentan notwendigen Anwendungszweck ausgebildet sind, verbunden werden. Zum anderen muss die Sonde nicht auf das Endoskop angepasst sein, mit welchem zusammen sie verwendet wird. Es kann z.B. die Sonde (wie meist üblich) sehr lang ausgebildet sein, so dass das zu ihr gehörige Gerät zur Versorgung mit HF-Strom sowie dem Inertgas in hinreichender Entfernung vom Operationsfeld aufgestellt werden kann, so dass keine Verlängerungskabel oder dergleichen notwendig sind. Dennoch kann die Griffeinrichtung ganz nahe an der Öffnung des Arbeitskanals fixiert werden, so dass keine Durchbiegung der Sonde beim Manipulieren, insbesondere beim Schieben möglich ist. Dadurch aber kann auch gleichzeitig die Griffeinrichtung als Anschlag dienen, so dass die Sonde nicht zu tief eingeführt werden kann und somit nicht zu weit aus dem distalen Ende des Endoskops heraussteht. Dies stellt somit auch einen Sicherheitsaspekt dar.

Bei einer bevorzugten Ausführungsform der Erfindung sind die Verbindungseinrichtungen derart ausgebildet, dass die Sonde an einem Abschnitt, der entfernt von ihren Enden, also dem distalen ebenso wie dem proximalen Ende liegt, während einer Benutzung der Sonde mit der Griffeinrichtung lösbar verbunden werden kann. Es kann somit die Griffeinrichtung bei Bedarf mit der Sonde verbunden werden, ohne dass ein umständliches "Einfädeln" in den Griff notwendig ist. Auch beim Wechseln von Sonden kann ein und dieselbe Griffeinrichtung von der auszutauschenden Sonde abgenommen und mit der neu einzusetzenden Sonde verbunden werden.

An den Griffeinrichtungen sind Schalteinrichtungen vorgesehen, die derart ausgebildet sind, dass insbesondere mit der Sonde verbundene medizinische Geräte durch die Schalteinrichtungen vom Benutzer steuerbar sind. Der Benutzer kann also mit den Fingern der Hand, mit welcher er die Sonde verschiebt, z.B. eine Absaugeinrichtung betätigen, insbesondere aber die Parameter steuern, mit welchen die Sonde betrieben wird, z.B. einen HF-Koagulationsstrom. Auf diese Weise kann ein Fußschalter entfallen, bzw. kann ein vorhandener Fußschalter für andere Zwecke verwendet werden. Insbesondere ist dadurch eine besonders logische Zuordnung der Betätigungseinrichtungen für die Geräte möglich, mit welchen zusammen die Sonde betrieben wird.

Vorzugsweise sind die Griffeinrichtung derart rotationssymmetrisch ausgebildet und mit der Sonde verbindbar, dass die Sonde ohne wesentliche Änderungen einer Griffposition des Benutzers um ihre Längsachse drehbar ist. Dies erleichtert die Handhabbarkeit des Sondenmanipulators erheblich.

Die Verbindungseinrichtungen können in verschiedener Art und Weise ausgebildet sein, z.B. Schraubeinrichtungen oder dergleichen umfassen. Bei einer bevorzugten Ausführungsform sind die Verbindungseinrichtungen jedoch als Klemmeinrichtungen ausgebildet, die vorzugsweise mit einer Hand bedienbar sind. Dadurch kann der Operateur während der Operation die Griffeinrichtung auf der Sonde verschieben oder sonstwie anders positionieren.

Bevorzugte Ausführungsformen ergeben sich aus den Unteransprüchen und der nachfolgenden Beschreibung bevorzugter Ausführungsformen der Erfindung, die anhand von Abbildungen näher erläutert werden. Hierbei zeigen
- - Fig. 1: eine perspektivische Ansicht zur Erläuterung einer ersten Ausführungsform nicht gemäß der Erfindung,
- - Fig. 2: eine perspektivische Ansicht einer Ausführungsform der Erfindung in einer Darstellung ähnlich der nach Fig. 1,
- - Fig. 3: einen Längsschnitt durch einen Sondenmanipulator mit eingeklemmtem Sondenschlauch,
- - Fig. 4: einen Schnitt entlang der Linie IV-IV aus Fig. 3 und
- - Fig. 5: einen Schnitt entlang der Linie V-V aus Fig. 3.

In der nachfolgenden Beschreibung werden für gleiche und gleich wirkende Teile dieselben Bezugszifferri verwendet.

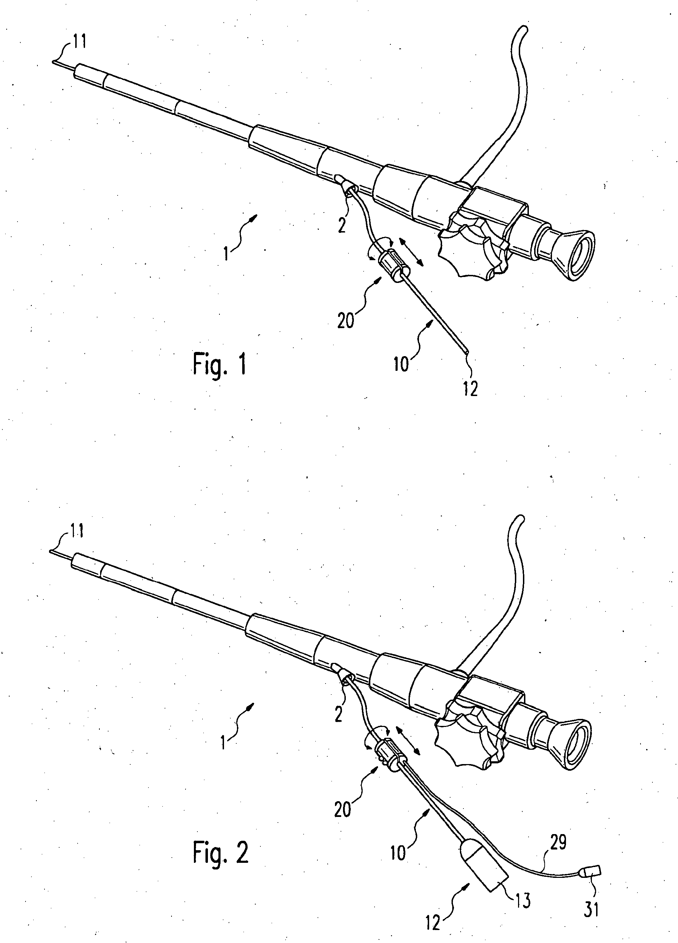

Gemäß Fig. 1 weist ein Endoskop 1 eine Öffnung 2 für einen darin (wie üblich) vorgesehenen Arbeitskanal auf. In diesen Arbeitskanal kann eine Sonde 10 derart eingeschoben werden, dass ihr distales Ende 11 aus dem Ende des Arbeitskanals hervorragt, ihr proximales Ende 12 jedoch so weit aus der Öffnung 2 des Arbeitskanals heraussteht, dass es an ein Betätigungsgerät, z.B. eine Quelle für eine Spülflüssigkeit, eine Saugeinrichtung oder ein Gerät für die Argon-Plasma-Koagulation anschließbar ist.

Auf die Sonde 10 ist nun eine Griffeinrichtung 20 derart aufsetzbar und mit der Sonde 10 verbindbar, dass eine Längsverschiebung der Sonde 10, also ein tieferes Einführen in den Arbeitskanal oder ein aus ihm Herausziehen sowie eine Drehung der Sonde innerhalb des Arbeitskanals (wie mit den Pfeilen in Fig. 1 angedeutet) ermöglicht wird.

Die in Fig. 2 gezeigte Ausführungsform der Erfindung unterscheidet sich von der nach Fig. 1 dadurch, dass die Griffeinrichtung 20 zusätzlich mit einem Verbindungskabel 29 verbunden ist, welches mittels eines Steckers 13 mit einem Gerät, insbesondere mit dem Gerät verbindbar ist, mit welchem die Sonde 10 betrieben wird. Darüber hinaus ist in Fig. 2 auch ein Stecker 13 gezeigt, über welchen das proximale Ende 12 der Sonde 10 an das für die Sonde vorgesehene Betätigungsgerät angeschlossen werden kann.

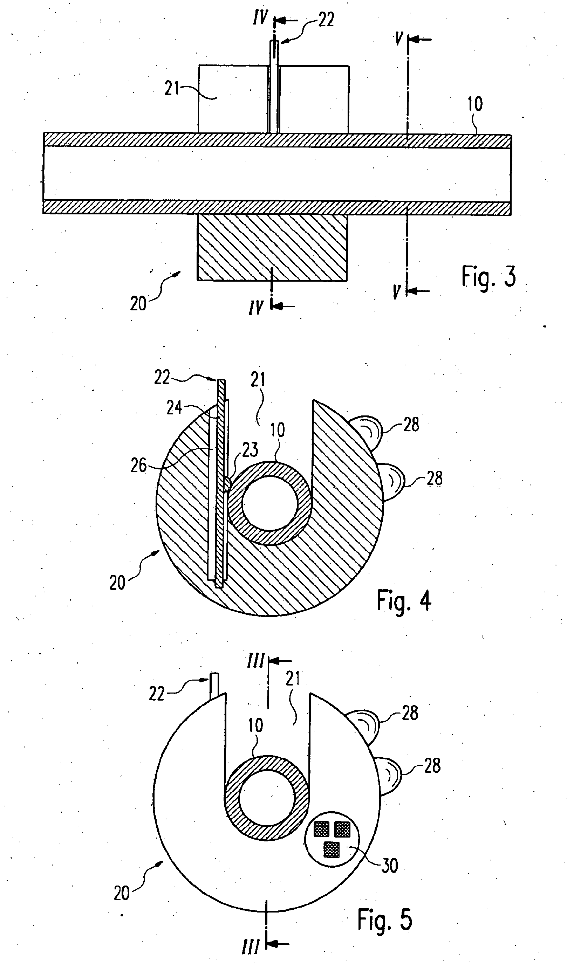

Nachfolgend wird die Griffeinrichtung 20 anhand der Fig. 3 - 5 näher erläutert. Bei der hier vereinfacht dargestellten Ausführungsform der Erfindung, welche der Griffeinrichtung nach Fig. 2 entspricht, ist die Griffeinrichtung 20 im Wesentlichen zylindrisch ausgebildet und weist einen Schlitz 21 auf, der einen, der Schlauchform der Sonde 10 angepassten Boden hat und so tief ausgebildet ist, dass die Sonde 10 im Zentrum der zylindrischen Griffeinrichtung 20 liegt.

Der Schlitz 21 ist derart nach außen offen, dass die Griffeinrichtung 20 über die gesamte Länge der Sonde 10 hinweg auf diese aufgeschoben werden kann, so dass man die Sonde 10 nicht mit einem ihrer Enden 11 oder 12 durch eine Öffnung hindurchfädeln muss.

Zum Verbinden der Griffeinrichtung 20 mit der Sonde 10 kann eine einfache Klemmeinrichtung genügen. Bei der hier gezeigten Ausführungsform der Erfindung besteht die Klemmeinrichtung aus einem Biegestab 24, der einen Klemmvorsprung 23 aufweist und aus einer Ausnehmung 26 der Griffeinrichtung 20 mit einem Klemmknopf 22 hervorsteht. Der Klemmknopf 22 kann (in Fig. 4 zur linken Seite, entgegen dem Uhrzeigersinn) verschoben werden, so dass sich der Biegestab 24 verbiegt und der Klemmvorsprung 23 die Sonde 10 freigibt. In diesem Zustand kann die Griffeinrichtung 20 entweder auf der Sonde 10 verschoben, verdreht oder ganz von der Sonde 10 abgenommen werden.

Weiterhin ist die Griffeinrichtung 20 bei der in den Fig. 2-5 gezeigten Ausführungsform mit Betätigungsschaltern 28 ausgestattet, welche über das Verbindungskabel 29 (dieses kann über einen Stecker mit einer Buchse 30 an der Griffeinrichtung verbunden werden) und den Stecker 31 mit einem Betätigungsgerät für die Sonde 10 verbunden sind. Damit kann also das Betätigungsgerät, z.B. ein HF-Generator mit einer AR-Quelle über die Betätigungsschalter 28 vom Operateur von der Hand gesteuert werden, mit welcher er die Griffeinrichtung hält und damit die Sonde 10 im Arbeitskanal des Endoskops 1 manipuliert.

### Bezugszeichenliste

- 1: Endoskop
- 2: Öffnung des Arbeitskanals
- 10: Sonde
- 11: Distales Ende
- 12: Proximales Ende
- 13: Stecker
- 20: Griffeinrichtung
- 21: Schlitz
- 22: Klemmknopf
- 23: Klemmvorsprung
- 24: Biegestab
- 26: Ausnehmung
- 28: Betätigungsschalter
- 29: Verbindungskabel
- 30: Buchse
- 31: Stecker

## Patentansprüche

1. Sondenmanipulator mit einer von einem Benutzer ergreifbaren Griffeinrichtung (20) zum Manipulieren einer Sonde (10), die in einen Arbeitskanal eines Endoskops (1) eingeführt ist,
wobei
eine Verbindungseinrichtung (21-24) zum lösbaren Verbinden der Sonde (10) mit der Griffeinrichtung (20) derart vorgesehen ist, dass die Sonde (10) beim Drehen oder Verschieben der Griffeinrichtung (20) gedreht bzw. verschoben wird, und wobei
an der Griffeinrichtung (20) Schalteinrichtungen (28) vorgesehen sind, die derart ausgebildet sind, dass mit der Sonde (10) verbundene medizinische Geräte durch die Schalteinrichtungen (28) vom Benutzer steuerbar sind.

2. Sondenmanipulator nach Anspruch 1,
**dadurch gekennzeichnet, dass**
die Verbindungseinrichtung (21-24) derart ausgebildet ist, dass die Sonde (10) an einem Abschnitt, der entfernt von ihren Enden (11, 12) liegt, während einer Benutzung der Sonde (10) mit der Griffeinrichtung (20) lösbar verbindbar ist.

3. Sondenmanipulator nach einem der vorhergehenden Ansprüche,
**dadurch gekennzeichnet, dass**
die Griffeinrichtungen (20) derart rotationssymmetrisch ausgebildet und mit der Sonde (10) verbindbar sind, dass die Sonde (10) ohne wesentliche Änderung einer Griffposition des Benutzers um ihre Längsachse drehbar ist.

4. Sondenmanipulator nach einem der vorhergehenden Ansprüche,
**dadurch gekennzeichnet, dass**
die Verbindungseinrichtung (21-24) als einhändig bedienbare Klemmeinrichtung ausgebildet ist.

## Claims

1. Probe manipulator with a grip device (20) that can be grasped by a user in order to manipulate a probe (10) that is inserted into a working channel of an endoscope (1), wherein a connection device (21-24) is provided for releasably connecting the probe (10) to the grip device (20) in such a way that the probe (10) is rotated or displaced upon respective rotation or displacement of the grip device (20), and wherein switch devices (28) are provided on the grip device (20) and are designed in such a way that medical appliances connected to the probe (10) can be controlled by the user via the switch devices (28).

2. Probe manipulator according to Claim 1, **characterized in that** the connection device (21-24) is designed in such a way that the probe (10) can be releasably connected to the grip device (20) at a section of the probe (10) lying at a distance from its ends (11, 12) during the use of the probe (10).

3. Probe manipulator according to one of the preceding claims, **characterized in that** the grip devices (20) are rotationally symmetrical, and connectable to the probe (10), in such a way that
the probe (10) is rotatable about its longitudinal axis without the user appreciably changing the gripping position.

4. Probe manipulator according to one of the preceding claims, **characterized in that** the connection device (21-24) is designed as a clamp device that can be operated with one hand.

## Revendications

1. Manipulateur de sonde comprenant un dispositif de préhension (20) pouvant être saisi par un utilisateur pour manipuler une sonde (10) qui est introduite dans un canal de travail d'un endoscope (1),
un dispositif de connexion (21-24) pour la connexion détachable de la sonde (10) au dispositif de préhension (20) étant prévu, de telle sorte que la sonde (10) soit tournée ou coulissée lors de la rotation ou du coulissement du dispositif de préhension (20), et
des dispositifs de commutation (28) étant prévus au niveau du dispositif de préhension (20), lesquels sont réalisés de telle sorte que des appareils médicaux connectés à la sonde (10) puissent être commandés par l'utilisateur au moyen des dispositifs de commutation (28).

2. Manipulateur de sonde selon la revendication 1, **caractérisé en ce que**
le dispositif de connexion (21-24) est réalisé de telle sorte que la sonde (10) puisse être connectée de manière détachable au dispositif de préhension (20) au niveau d'une portion qui est éloignée de ses extrémités (11, 12), pendant une utilisation de la sonde (10).

3. Manipulateur de sonde selon l'une quelconque des revendications précédentes,
**caractérisé en ce que**
les dispositifs de préhension (20) sont réalisés avec une symétrie de révolution et peuvent être connectés à la sonde (10) de telle sorte que la sonde (10) puisse tourner autour de son axe longitudinal sans modification essentielle d'une position de préhension de l'utilisateur.

4. Manipulateur de sonde selon l'une quelconque des revendications précédentes,
**caractérisé en ce que**
le dispositif de connexion (21-24) est réalisé sous forme de dispositif de serrage pouvant être manipulé d'une seule main.
